(19) Europäisches Patentamt
European Patent Office
Office européen des brevets

(11) EP 4 172 161 B1

(12) # FASCICULE DE BREVET EUROPEEN

(45) Date de publication et mention de la délivrance du brevet:
**18.03.2026 Bulletin 2026/12**

(21) Numéro de dépôt: **21736246.6**

(22) Date de dépôt: **25.06.2021**

(51) Classification Internationale des Brevets (IPC):
***C07D 493/04*** (2006.01) ***C08K 5/1535*** (2006.01)
***C08G 59/26*** (2006.01) ***C08L 37/00*** (2006.01)

(52) Classification Coopérative des Brevets (CPC):
**C07D 493/04; C08G 59/04; C08G 59/226; C08G 59/26**

(86) Numéro de dépôt international:
**PCT/EP2021/025235**

(87) Numéro de publication internationale:
**WO 2022/002438 (06.01.2022 Gazette 2022/01)**

(54) **PROCÉDÉ D'OBTENTION DE POLYÉPOXYDES BIOSOURCÉS AUX PROPRIÉTÉS AMÉLIORÉES**

VERFAHREN ZUR HERSTELLUNG VON POLYEPOXIDEN BIOLOGISCHEN URSPRUNGS MIT VERBESSERTEN EIGENSCHAFTEN

METHOD FOR OBTAINING BIOSOURCED POLYEPOXIDES WITH IMPROVED PROPERTIES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.06.2020 FR 2006915**

(43) Date de publication de la demande:
**03.05.2023 Bulletin 2023/18**

(73) Titulaire: **ROQUETTE FRERES**
**62136 Lestrem (FR)**

(72) Inventeurs:
- **VANBESIEN, Théodore**
**59280 Armentières (FR)**
- **AMEDRO, Hélène**
**62400 Béthune (FR)**
- **SAHUT, Audrey**
**62136 Lestrem (FR)**
- **SAINT-LOUP, René**
**59160 Lomme (FR)**

(74) Mandataire: **Plasseraud IP**
**104 Rue de Richelieu**
**CS92104**
**75080 Paris Cedex 02 (FR)**

(56) Documents cités:
**EP-A1- 2 885 305** **EP-A1- 3 197 899**
**WO-A1-2011/029548** **KR-A- 20160 123 563**
**US-A1- 2005 234 121** **US-A1- 2018 230 261**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

### Domaine technique

La présente divulgation relève du domaine des polyépoxydes et concerne en particulier un procédé de préparation de prépolymères époxy comprenant des glycidyléthers de motifs dianhydrohexitol ainsi que des glycidyléthers d'autres motifs alcool.

### Technique antérieure

Les polyépoxydes, encore appelés polymères époxydes ou communément « époxy » sont largement utilisés, aussi bien en tant que matériau de surface, par exemple pour la fabrication d'adhésifs ou de revêtements, qu'en tant que matériaux de structure, par exemple comme matrice de matériaux composites.

Les polyépoxydes sont obtenus par durcissement de compositions durcissables comprenant des prépolymères époxy.

Au sens de la présente invention, un prépolymère époxy est un mélange de molécules comprenant des fonctions époxyde capable de subir une polymérisation ultérieure conduisant à l'obtention d'un polyépoxyde. Les prépolymères époxy peuvent comprendre ou non une fraction oligomérique. Ils peuvent comprendre ou non une fraction polymérique.

La plupart des compositions durcissables comprenant des prépolymères époxy utilisées, notamment, pour la fabrication d'adhésifs, de revêtements, ou de matrices pour des matériaux composites contiennent, outre des prépolymères époxy, au moins un durcisseur et/ou au moins un accélérateur.

Lors du durcissement de la composition durcissable comprenant des prépolymères époxy, des liaisons chimiques sont formées entre molécules du prépolymère époxy et/ou entre le prépolymère époxy et un durcisseur par des réactions d'ouverture des fonctions époxyde du prépolymère époxy. On aboutit ainsi à la formation d'un réseau macromoléculaire tridimensionnel.

Par accélérateur, on entend des composés permettant de catalyser la réaction d'homopolymérisation entre deux fonctions époxyde ou la réaction entre une fonction époxyde et l'agent durcisseur. Les acides de Lewis, les bases de Lewis et les photoinitiateurs en sont des exemples.

Par durcisseur on entend tout composé différent des prépolymères époxy permettant de former un réseau tridimensionnel par réaction avec les fonctions époxyde desdits prépolymères. Les amines, les amidoamines, les bases de Mannich, les acides organiques (dont les polyesters terminés par des fonctions carboxyliques), les anhydrides d'acides organiques, les durcisseurs latents (type cyanamide, imidazole,...) en sont des exemples.

Dans les compositions durcissables mono-composantes, les accélérateurs et/ou durcisseurs sont directement incorporés dans la composition de prépolymères époxy : on parle de systèmes 1K. Dans les compositions durcissables bi-composantes, l'agent accélérateur et/ou l'agent durcisseur est conditionné séparément de la composition de prépolymères époxy et le mélange n'intervient qu'au moment de l'application de la mise en forme de la composition durcissable : on parle de systèmes 2K.

Les compositions durcissables comprenant des prépolymères époxy peuvent également contenir des charges organiques ou inorganiques (silice, sable, oxyde d'aluminium, talc, carbonate de calcium...), des pigments, des plastifiants, des stabilisants, des agents thixotropes.

Le Diglycidyléther de Bisphénol A (BADGE ou DGEBA), de formule (i), est un composé chimique aujourd'hui très largement utilisé comme prépolymère époxy.

[Chem. 1]

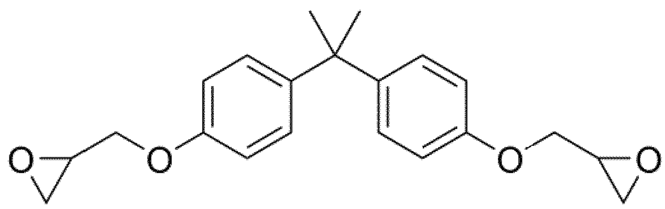

(i)

Le DGEBA est un produit obtenu à partir du pétrole ce qui est un inconvénient dans un contexte de renchérissement et/ou de raréfaction des ressources pétrolières.

D'autre part, le bisphénol A est aujourd'hui reconnu comme perturbateur endocrinien.

Ceci rend potentiellement dangereux pour la santé la manipulation de prépolymères époxy à base de bisphénol A ou le contact avec les polyépoxydes obtenus partir du DGEBA.

Il est connu depuis quelques années que le DGEBA peut être remplacé par des mélanges contenant du diglycidyléther d'isosorbide, qui est un produit biosourcé dont la structure est représentée ci-dessous (formule (ii)).

[Chem. 2]

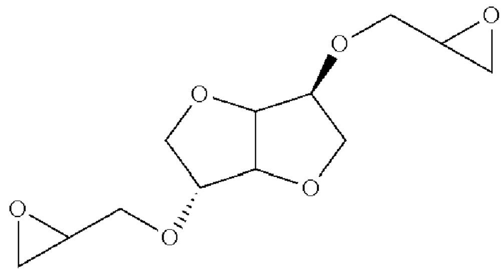

(ii)

Ce composé, qui appartient à la classe plus générale des diglycidyléthers de dianhydrohexitols, est aujourd'hui largement connu et décrit dans la littérature, de même que son procédé de synthèse. On citera, par exemple, les documents US3272845, US4770871, WO2008/147472, WO2008/147473, US3041300, WO2012/157832 et WO2015/110758 qui divulguent des procédés de synthèse de diglycidyléthers de dianhydrohexitols.

Lorsqu'on met en œuvre l'un quelconque des procédés décrits dans les documents cités précédemment, on obtient en fait une composition de prépolymères époxy contenant, en plus du diglycidyléther de dianhydrohexitol, le monoglycidyléther de dianhydrohexitol ainsi que des oligomères comprenant des motifs dianhydrohexitol et glycéryl, Ces oligomères peuvent comporter un ou plusieurs groupements glycidyléther portés par des motifs dianhydrohexitol et/ou des motifs glycéryl.

Dans la présente demande, on nomme « prépolymère époxy à base d'un alcool » un prépolymère époxy dans lequel les fonctions époxyde sont essentiellement incluses dans des groupements glycidyléther et dans lequel, les groupements glycidyléther sont essentiellement portés par des motifs ledit alcool ou des motifs glycéryl qui eux-mêmes sont liés à des motifs ledit alcool.

Par exemple, dans un prépolymère époxy à base d'isosorbide, les groupements glycidyléthers sont essentiellement portés par des motifs isosorbide et des motifs glycéryl liés à des motifs isosorbide. Les groupements glycidyléther se présentent ainsi par exemple sous forme de mono- ou di- glycidyléther d'isosorbide ou sous forme de motifs glycidyléther-isosorbide-... dans des oligomères.

Ainsi, les compositions de prépolymères époxy obtenues en mettant en œuvre les procédés décrits dans les documents cités précédemment sont des prépolymères époxy à base d'un dianhydrohexitol ou à base d'isosorbide.

Dans la présente demande, on nomme « polyépoxyde à base d'un alcool » un polyépoxyde obtenu par durcissement d'un prépolymère époxy à base dudit alcool.

La présence de composés monoglycidyléther et/ou d'oligomères en plus de composés diglycidyléther dans un prépolymère époxy à base d'un diol diminue la densité de réticulation dans le réseau macromoléculaire tridimensionnel obtenu par durcissement d'une composition durcissable comprenant ledit prépolymère époxy par rapport à ce qui serait obtenu si la composition durcissable comprenait le diglycidyléther dudit diol pur comme prépolymère époxy.

Cette densité de réticulation est à relier à la température de transition vitreuse (Tg) du polyépoxyde. Une densité de réticulation importante permet d'obtenir un matériau ayant une température de transition vitreuse (Tg) plus élevée et plus résistant chimiquement et mécaniquement.

La présence d'oligomères et/ou de mono-glycidyléthers dans un prépolymère époxy peut être directement reliée à l'équivalent d'époxy en poids (EEW), défini comme la masse de prépolymère époxy contenant un équivalent de groupements glycidyléther Par exemple, du diglycidyléther d'isosorbide pur (formule ii), qui a un poids moléculaire de 258 g/mol et qui contient 2 groupements glycidyléther, possède un équivalent époxy de 129 g/eq.

Dans un prépolymère époxy à base d'un diol, l'EEW est minimal si ledit prépolymère époxy est du diglycidyléther dudit diol pur. L'EEW dudit prépolymère époxy augmente quand la teneur en oligomère et/ou en mono-glycidyléther dudit diol augmente dans ledit prépolymère époxy.

Des polyépoxydes ont été préparés à partir de prépolymères époxy à base de diglycidyléther d'isosorbide.

Il reste cependant difficile d'obtenir des polyépoxydes biosourcés présentant des performances équivalentes aux polyépoxydes obtenus à partir de composés pétrosourcés tels que le DGEBA.

Le document US2015/0353676 A1 décrit notamment des polyépoxydes à base d'isosorbide et, en tant que durcisseur, d'acide cis-4-cyclohexène-1,2-dicarboxylique.

Le document US2018/0230261 A1 décrit des polyépoxydes à base d'isosorbide et, en tant que durcisseur, d'un polyamide.

Le document WO2015/110758 A1 décrit des polyépoxydes à base d'isosorbide et, en tant que durcisseur,

d'isophorone diamine. Ces polyépoxydes présentent des températures de transition vitreuse de l'ordre de 95-100°C.

De même, les documents US2017/0253692 et JP2014189713 décrivent des polyépoxydes à base d'isosorbide comprenant divers durcisseurs.

Un problème récurrent rencontré jusqu'ici avec les polyépoxydes à base d'isosorbide est leur reprise en eau importante, c'est-à-dire que les molécules d'eau ont de la facilité à diffuser dans le réseau macromoléculaire tridimensionnel du polyépoxyde. Ainsi, en présence d'humidité ou d'eau liquide, ces polyépoxydes ont tendance à se charger en eau, ce qui a notamment pour conséquences de plastifier le polyépoxyde (diminution de sa température de transition vitreuse), de le faire gonfler et de diminuer ses propriétés de cohésion ou d'adhésion.

Une reprise en eau importante n'est donc pas compatible avec de nombreuses applications des polyépoxydes (adhésifs, matrice pour matériaux composites notamment) ce qui est actuellement un des principaux freins à une utilisation large des polyépoxydes à base de dianhydrohexitol.

Une solution à ce problème, développée par la société demanderesse, consiste à préparer des polyépoxydes à partir de mélanges entre prépolymères époxy à base de dianhydrohexitols et prépolymères époxy à base d'autres alcools.

L'objectif est de combiner les propriétés des polyépoxydes à base de dianhydohexitols et les propriétés des polyépoxydes à base des autres alcools tout en compensant leurs défauts respectifs.

Poursuivant ses recherches, la société Demanderesse a cependant trouvé que si le dianhydrohexitol et l'autre alcool sont mélangés avant l'étape de synthèse du prépolymère époxy, on obtient des propriétés meilleures, notamment des reprises en eau plus faibles pour le polyépoxyde qui en résulte, que pour le polyépoxyde obtenu à partir du mélange des prépolymères époxy synthétisés séparément à partir du dianhydrohexitol et de l'autre alcool. Cet effet surprenant est l'objet de la présente invention.

## Résumé

La présente divulgation concerne la préparation de prépolymères époxy comprenant des glycidyléthers de dianhydrohexitols permettant d'obtenir des polyépoxydes à reprise en eau améliorée.

Il est ainsi proposé un procédé de préparation d'une composition de prépolymères époxy comprenant des glycidyléthers, ledit procédé comprenant les étapes suivantes :

a. Mettre en contact un dianhydrohexitol et un autre alcool de manière à obtenir une composition d'alcools ;

b. Faire réagir la composition d'alcools obtenue à l'étape a) avec une épihalohydrine de manière à obtenir un mélange réactionnel comprenant des glycidyléthers ;

c. Récupérer la composition de prépolymères époxy comprenant des glycidyléthers à partir du mélange réactionnel obtenu à l'issue de l'étape b).

Selon un autre aspect, il est proposé une composition de prépolymères époxy susceptible d'être obtenue par le procédé selon l'invention.

Selon un autre aspect, il est proposé une composition durcissable comprenant une composition de prépolymères époxy selon l'invention, caractérisée en ce qu'elle comprend en outre au moins un accélérateur et/ou au moins un durcisseur.

Selon un autre aspect, il est proposé un polyépoxyde obtenu par durcissement d'une composition durcissable selon l'invention.

Selon un autre aspect, il est proposé un matériau composite, un revêtement ou un adhésif comprenant un polyépoxyde selon l'invention.

D'autres caractéristiques et avantages de la présente invention apparaîtront à la lecture de la description détaillée suivante.

## Description détaillée

Dans la présente demande de brevet, l'expression « compris(e) entre ... et ... » doit être entendue comme incluant les bornes.

Il est proposé un procédé de préparation d'une composition de prépolymères époxy comprenant des glycidyléthers, ledit procédé comprenant les étapes suivantes :

a) Mettre en contact un dianhydrohexitol et un autre alcool de manière à obtenir une composition d'alcools ;
b) Faire réagir la composition d'alcools obtenue à l'étape a) avec une épihalohydrine de manière à obtenir un mélange réactionnel comprenant des glycidyléthers ;

c) Récupérer la composition de prépolymères époxy comprenant des glycidyléthers à partir du mélange réactionnel obtenu à l'issue de l'étape b).

La première étape du procédé selon l'invention (étape a) consiste donc à mettre en contact un dianhydrohexitol et un autre alcool.

La mise en contact entre le dianhydrohexitol et l'autre alcool peut se faire en mélangeant les deux composés, par exemple par mise en solution du dianhydrohexitol et de l'autre alcool ou par fusion du dianhydrohexitol et de l'autre alcool dans le cas où ils sont miscibles à l'état liquide, ou par mélange de ces composés à l'état solide, par exemple sous forme de poudre.

Les dianhydrohexitols sont des composés hétérocycliques obtenus par double déshydratation d'hexitols (iditol, mannitol ou sorbitol, par exemple). Ce sont donc des diols. Parmi les dianhydrohexitols, les isohexides correspondent aux 1,4:3,6-dianhydrohexitols et comprennent l'isosorbide, l'isoidide et l'isomannide.

Dans le procédé selon l'invention, le dianhydrohexitol est de préférence un isohexitol, plus préférentiellement choisi parmi l'isosorbide, l'isomannide et l'isoidide, et est, le plus préférentiellement, l'isosorbide.

Dans le procédé selon l'invention, l'étape b. de réaction avec une épihalohydrine permet la transformation de fonctions alcools du dianhydrohexitol et de l'autre alcool en groupements glycidyléther. A cette étape peuvent aussi se former des oligomères. Parmi ces oligomères, certains comprennent des motifs dianhydrohexitol ou des motifs autre alcool et certains comprennent un mélange de motifs dianhydrohexitol et de motifs autre alcool.

Le mélange réactionnel à l'issue de l'étape b. contient donc une composition de prépolymères époxy comprenant des groupements glycidyléthers portés par des motifs dianhydrohexitol et des groupements glycidyléther portés par des motifs autre alcool.

Le fait de mélanger au moins un dianhydrohexitol et au moins un autre alcool à l'étape a., c'est-à-dire préalablement à l'étape b. de réaction avec l'épihalohydrine résulte en un effet inattendu.

En effet, les polyépoxydes obtenus par durcissement de compositions durcissables comprenant des compositions de prépolymères époxy obtenues par le procédé selon l'invention présentent des propriétés, par exemple des propriétés de reprise en eau, améliorées par rapport aux propriétés de polyépoxydes obtenus par durcissement de compositions durcissables comparables résultant du mélange d'un prépolymère époxy à base du dianhydrohexitol et d'un prépolymère époxy à base de l'autre alcool.

Sans vouloir limiter la portée de l'invention à une quelconque théorie, on peut penser que cette amélioration inattendue du polyépoxyde soit liée à la présence d'oligomères comprenant à la fois des motifs dianhydrohexitol et des motifs autre alcool dans la composition de prépolymères époxy obtenue par le procédé de l'invention.

Dans le procédé selon la présente invention, l'autre alcool mis en contact avec un dianhydrohexitol à l'étape a. n'est de préférence pas un dianhydrohexitol.

Plus préférentiellement, l'autre alcool mis en contact avec un dianhydrohexitol à l'étape a. est choisi parmi les alcools suivants :

- Triméthylol éthane,
- Triméthylol propane,
- Spiroglycol,
- Tricyclodécanediméthanol,
- Glycérol,
- Hexan-1,6-diol,
- Diols aliphatiques en $C_n$, où $n \geq 7$,
- Cyclohexan-1,m-diméthanol, où m = 2, 3 ou 4,
- Furan-p,q-diméthanol, où {p,q} = {1,4}, {1,3} ou {2,3},
- Thiophen-p,q-diméthanol, où {p,q} = {1,4}, {1,3} ou {2,3},
- Isobornéol
- Dodécanol, ou
- Décanol.

Selon un mode de réalisation du procédé selon l'invention l'autre alcool comprend au moins deux fonctions alcool.

Si l'autre alcool ne comprend qu'une fonction alcool, il ne peut être intégré qu'en bout de chaine dans les oligomères comprenant à la fois des motifs dianhydrohexitol et des motifs autre alcool contenus dans la composition de prépolymères époxy obtenue par le procédé de l'invention. Si l'autre alcool comprend au moins deux fonctions alcool, la composition de prépolymères époxy obtenue par le procédé de l'invention peut comprendre des oligomères comprenant des motifs ...-dianhydrohexitol-glycéryl-autre alcool-glycéryl-... ou des motifs ...-dianhydrohexitol-glycéryl-autre alcool-glycidyl.

Avantageusement, l'autre alcool mis en contact avec un dianhydrohexitol à l'étape a. du procédé selon l'invention est choisi de telle manière qu'un polyépoxyde à base dudit autre alcool présente une reprise en eau plus faible que celle d'un polyépoxyde à base dudit dianhydrohexitol.

En d'autres termes, un polyépoxyde obtenu par un procédé de durcissement d'une composition de prépolymères époxy obtenue par un procédé d'éthérification de l'autre alcool avec une épihalohydrine, a de préférence une reprise en eau inférieure à la reprise en eau, mesurée selon la même méthode, d'un polyépoxyde obtenu par les mêmes procédés de durcissement et d'éthérification mais en remplaçant l'autre alcool par le dianhydrohexitol.

Dans le procédé selon l'invention, l'autre alcool est de préférence le 1,4-cyclohexanediméthanol (CHDM).

Dans le procédé selon l'invention, le ratio r entre le nombre de moles du dianhydrohexitol et la somme du nombre de moles de l'autre alcool et du nombre de moles du dianhydrohexitol ($r = n_{dianhydrohexitol}/(n_{dianhydrohexitol} + n_{autre\ alcool})$) est de préférence compris entre 0,05 et 0,95, plus préférentiellement entre 0,1 et 0,9.

Dans le procédé selon l'invention, l'épihalohydrine est de préférence choisie parmi l'épibromohydrine, l'épifluorohydrine, l'épiiodohydrine et l'épichlorhydrine, et est, plus préférentiellement, l'épichlorhydrine.

Dans le procédé selon l'invention, l'étape b. de réaction avec épihalohydrine permet la transformation de fonctions alcools du dianhydrohexitol et de l'autre alcool en groupements glycidyléther.

Elle peut se réaliser selon tout procédé connu de l'homme du métier permettant la transformation de fonctions alcools du dianhydrohexitol et de l'autre alcool en groupements glycidyléther, par exemple, les procédés décrits dans les documents US3272845, US4770871, WO2008/147472, WO2008/147473, US3041300, WO2012/157832 et WO2015/110758, de préférence le procédé décrit dans le document WO2015/110758.

Ainsi, dans le procédé selon l'invention, l'étape b) de réaction de la composition d'alcools avec l'épihalohydrine comprend de préférence les étapes suivantes :

b1) Mettre en contact la composition d'alcools et l'épihalohydrine de manière à obtenir un mélange réactionnel ;
b2) Placer le mélange réactionnel obtenu à l'étape b1) sous vide de manière à obtenir une dépression comprise entre 100mbar et 1000mbar ;
b3) Chauffer le mélange réactionnel obtenu à l'étape b2), en maintenant ladite dépression, à une température comprise entre 50°C et 120°C de manière à réaliser une distillation de l'épihalohydrine ;
b4) Ajouter au mélange réactionnel obtenu à l'étape b3) un réactif basique pendant une durée comprise entre 1h et 10h tout en maintenant le mélange réactionnel à ladite dépression et à ladite température de manière à réaliser une distillation azéotropique de l'azéotrope eau-épihalohydrine.

L'étape b1) de mise en contact de la composition d'alcools et de l'épihalohydrine est effectuée dans tout dispositif bien connu de l'homme du métier, permettant de réaliser la mise en contact entre des réactifs chimiques, et étant muni d'organes de chauffage et d'agitation. Il peut s'agir, à titre d'exemple, d'un réacteur double enveloppe. Le dispositif en question doit également être équipé d'un organe permettant de réaliser un vide partiel et d'un organe permettant de conduire une distillation azéotropique, tel qu'un montage Dean-Stark inversé surmonté d'un réfrigérant.

L'épihalohydrine est préférentiellement introduite en excès par rapport aux fonctions hydroxyles des alcools présents dans la composition d'alcools (comprenant le dianhydrohexitol et l'autre alcool). Ainsi, pour 1 mole de fonctions hydroxyles on introduira préférentiellement entre 1 et 5 moles d'épihalohydrine et plus préférentiellement environ 2,5 moles d'épihalohydrine.

Après cette première étape de mise en contact (étape b1), on réalise un vide partiel dans le dispositif à l'aide d'une pompe à vide, la dépression correspondante étant comprise entre 100 mbar et 1000 mbar (étape b2). Ceci signifie que la pression dans le réacteur est égale à la différence entre la pression atmosphérique (1013 mbar) et la dépression (entre 100 mbar et 1000 mbar), soit une pression dans le réacteur comprise entre 13 mbar et 913 mbar.

Selon un mode de réalisation, l'étape b2 est réalisée de manière à obtenir une dépression comprise entre 300 et 900 mbar, en particulier entre 500 et 800 mbar.

Au cours de l'étape b3), on chauffe le mélange entre la composition d'alcools et l'épihalohydrine à une température comprise entre 50°C et 120°C.

De préférence, la température de consigne de l'organe de chauffage du réacteur doit être réglée de manière à être au moins égale à la température d'ébullition de l'épihalohydrine utilisée, de manière à commencer la distillation de l'épihalohydrine. La température d'ébullition qu'il convient de prendre en compte est la température d'ébullition de l'épihalohydrine à la pression qui règne dans le réacteur.

Au cours de cette première phase de distillation, ladite distillation ne concerne que l'épihalohydrine. De plus, seule une partie de l'épihalohydrine est distillée. Cette partie distillée, peut par exemple être récupérée au niveau d'un Dean Stark inversé et éventuellement réintroduite dans le mélange réactionnel.

A titre d'exemple, l'épichlorhydrine présente une température d'ébullition de 116°C à pression atmosphérique, cette température d'ébullition étant environ égale à 80°C lorsque la pression dans le réacteur est de 275 mbar (ce qui correspond à une dépression de 738 mbar). De manière pratique, on règlera la température de consigne de l'organe de

chauffage du réacteur à une température légèrement supérieure (environ 30°C de plus) à la température d'ébullition pour l'épihalohydrine considérée et pour la dépression imposée.

Au cours de l'étape b4), on ajoute au mélange composition d'alcools / épihalohydrine un réactif basique, pendant une durée comprise entre 1 heure et 10 heures.

La quantité de réactif basique est préférentiellement la quantité stœchiométrique par rapport au nombre de fonctions hydroxyles des alcools présents dans la composition d'alcools. On peut néanmoins choisir de se placer en léger excès par rapport à cette stœchiométrie.

Le réactif basique est choisi de préférence parmi les hydroxydes de lithium, potassium, calcium ou de sodium, de préférence sous forme d'une solution aqueuse, et est, plus préférentiellement, une solution aqueuse d'hydroxyde de sodium.

Le rapport du nombre de moles d'$OH^-$ introduit avec le réactif basique sur le nombre de moles de fonctions hydroxyles des alcools présents dans la composition d'alcool est alors de préférence compris entre 0.9 et 1.2.

Dès l'introduction du réactif basique (étape b4), il y a formation d'eau par réaction entre la composition d'alcools et l'épihalohydrine, de même qu'il peut y avoir apport d'eau supplémentaire par introduction du réactif basique sous forme de solution aqueuse. La distillation est alors une distillation azéotropique qui concerne le mélange eau et épihalohydrine. Autrement dit, on distille l'azéotrope eau-épihalohydrine. Après décantation de l'azéotrope distillé, l'eau est éliminée et l'épihalohydrine retourne dans le milieu réactionnel. Dans le cas d'un dispositif Dean-Stark inversé : l'eau constitue la phase supérieure qui est éliminée, alors que l'épihalohydrine en partie inférieure est réintroduite dans le milieu réactionnel.

La distillation azéotropique est poursuivie de préférence jusqu'à élimination complète de l'eau. Ainsi le milieu réactionnel est encore chauffé pendant une durée comprise entre 30 minutes et 1 heure après la fin de l'addition du réactif basique.

De préférence, un catalyseur de transfert de phase est ajouté lors de l'étape b1). On parvient ainsi à diminuer de manière sensible la viscosité des produits fabriqués, tout en maintenant une proportion très importante de diglycidyléther du dianhydrohexitol par rapport au monoglycidyléther du dianhydrohexitol.

Le catalyseur de transfert de phase est préférentiellement choisi parmi les halogénures, sulfates ou hydrogénosulfates de tétra-alkylammonium et plus préférentiellement parmi le bromure de tétrabutylammonium ou l'iodure de tétrabutylammonium.

La quantité de catalyseur de transfert de phase est de préférence comprise entre 0,01 et 5%, plus préférentiellement entre 0,1% et 2% et encore plus préférentiellement est de 1% en poids par rapport au total représenté par la somme des masses du dianhydrohexitol et de l'autre alcool. On parvient alors à réduire très notablement l'EEW de la composition de prépolymères époxy obtenue.

De préférence, l'étape c) comprend une étape de filtration du milieu réactionnel obtenu à l'issu de l'étape b) de manière à obtenir un filtrat comprenant la composition de prépolymères époxy. Cette étape de filtration permet d'éliminer les sels formés au cours de la réaction entre l'épihalohydrine et la composition d'alcools, comme le chlorure de sodium dans le cas de l'épichlorhydrine. Les sels séparés par filtration sont de préférence lavés une nouvelle fois avec de l'épihalohydrine. L'épihalohydrine de lavage combinée au premier filtrat constitue alors le filtrat comprenant la composition de prépolymères époxy.

L'étape de filtration (incluant le lavage des sels éliminés) est de préférence suivie d'une étape de concentration du filtrat et/ou de purification du filtrat.

L'étape de concentration peut permettre, par exemple, d'éliminer l'épihalohydrine non réagie et/ou l'épihalohydrine de lavage. Elle peut se faire, par exemple, par distillation sous vide, par exemple dans un dispositif de type rotavapor et/ou évaporateur film raclée. Lors de cette étape de concentration, le produit brut ou la composition de prépolymères époxy est par exemple chauffé progressivement jusqu'à 140°C et la pression est par exemple diminuée jusqu'à 1 mbar (correspondant à une dépression de 1012 mbar).

L'étape de purification peut se faire, par exemple, par distillation sous pression réduite (pression<1mbar correspondant à une dépression>1012 mbar) et peut être réalisée au moyen d'un échangeur à surface raclée afin de séparer des composés oligomères des composés diglycidyléther de dianhydrohexitol ou de l'autre alcool. Cette étape est distincte de celle décrite dans le paragraphe précédent.

Selon un autre aspect de la présente invention il est proposé une composition de prépolymères époxy susceptible d'être obtenue par le procédé selon l'invention.

Les compositions de prépolymère époxy susceptibles d'être obtenues par le procédé selon l'invention présentent l'avantage d'avoir une viscosité réduite par rapport aux prépolymères époxy à base de dianhydrohexitol obtenus par le même procédé mais sans ajouter un autre alcool au dianhydrohexitol.

Avantageusement les compositions de prépolymère époxy susceptibles d'être obtenues par le procédé selon l'invention présentent une viscosité Brookfield, mesurée à 25°C, inférieure à 4000 mPa.s, de préférence inférieure à 1000 mPa.s, sans nécessiter d'étape de purification par séparation des composés oligomères des composés diglycidyléther.

Une faible viscosité facilite la mise en forme et la processabilité des prépolymères époxy. Par exemple, la

fabrication par coulage, enrobage, infusion, imprégnation, stratification, injection, pultrusion, ou par enroulement filamentaire de matériaux composites comprenant un polyépoxyde en tant que matrice est facilitée. Une faible viscosité permet également par exemple de faciliter les dépôts de couches minces, l'utilisation d'un pistolet, d'un rouleau lors de la mise en œuvre de polyépoxydes en tant que revêtements ou adhésifs.

La viscosité est mesurée à l'aide d'un viscosimètre rotatif Brookfield, type DV-II+. La mesure est effectuée après stabilisation du milieu maintenu à 25°C à l'aide d'un bain d'eau thermostaté. Les mesures de viscosité sont obtenues avec un couple (Torque) en % du couple maximum du viscosimètre compris entre 10 et 100%.

Dans toute la présente Demande, la vitesse à laquelle est déterminée la viscosité Brookfield n'est pas indiquée. L'homme du métier sait en effet l'adapter par rapport au choix du rotor et de manière à se placer à un pourcentage du couple (Torque) maximum du viscosimètre compris entre 10 et 100%.

Une fois préparée, la composition de prépolymères époxy selon l'invention peut être réticulée pour former un polyépoxyde durci. Il peut être préférable d'ajouter un durcisseur et/ou un accélérateur pour initier ou accéler la réticulation.

Selon un autre aspect de la présente invention, il est proposé une composition durcissable comprenant une composition de prépolymères époxy selon l'invention, caractérisée en ce qu'elle comprend en outre au moins un accélérateur et/ou au moins un durcisseur.

On entend par « composition durcissable », un mélange liquide qui est capable de polymériser pour former une résine réticulée (durcie). Par exemple, une composition durcissable comprenant des prépolymères époxy est un mélange liquide capable de polymériser pour former un polyépoxyde, qui est par définition une résine réticulée.

De préférence, la composition durcissable comprend un durcisseur de type amine

Le durcisseur de type amine peut, par exemple, être choisi parmi:

- les diamines linéaires aliphatiques, en particulier le 1,2-diaminométhane, le 1,3-diaminopropane, le butane-1,4-diamine, le pentane-1,5-diamine, le 1,6-diaminohexane, ou le 1,12-diaminododecane,
- des diamines cycliques aliphatiques, en particulier l'isophorone diamine (IPDA), le 4,4'-diaminodicyclohexylméthane (PACM), le 1,2-diaminocyclohexane (DACH), le menthanediamine, ou le 1,3-bis(aminomethyl)cyclohexane (1,3 BAC),
- les diamines aromatiques, en particulier la 4,4'-méthylènebis(2 aminophényl)fluorène (BAFL), le diéthyltoluène diamine (DETDA), le diméthyl aminophényl)fluorène (BAFL), le diéthyltoluène diamine (DETDA), le diméthyl thiotoluène diamine (DMTDA), le 4,4'-methylenebis(2-ethylaniline) (MOEA), le m-xylènediamine, le m-phénylènediamine (MPDA), ou le 4,4'-diaminodiphénylméthane,
- les triamines, en particulier le diéthlyène triamine (DTA),
- les tetramines, en particulier le triéthylènetetramine,
- les pentamines, en particulier le tetraéthylènepentamine,
- les diamines d'acide gras dimériques, en particulier la Priamine ® 1074 de Croda,
- les polyétheramines, en particulier le poly(oxypropylène) diamine (Jeffamine® D-230 de Huntsman Petrochemical, LLC), ou le poly(oxypropylène) triamine (Jeffamine® T-403 de Huntsman Petrochemical, LLC), ou
- tout autre polyamine, en particulier la polyethylene imine (e.g. Lupasol ® FG de BASF), le dipropènediamine, le diéthylaminopropylamine, le N-aminoéthylpipérazine, le dicyandiamide (Dicy),
- ou un mélange de celles-ci.

De préférence, le durcisseur est l'isophorone diamine.

Le système époxy/amine que forme la composition durcissable selon l'invention peut être stœchiométrique ou contenir un excès de fonctions amines ou un excès de fonctions époxy.

Le rapport du nombre de liaisons N-H dans la formule du durcisseur (D) sur le nombre de groupements époxy de la composition de prépolymères époxy peut être ainsi compris entre 1:2 et 2:1, en particulier entre 2:3 et 3:2, plus particulièrement être égal à 1:1 (mélange stœchiométrique).

Par exemple une fonction amine primaire comprend deux liaisons N-H. Ainsi on comptera 4 liaisons N-H par molécule d'isophorone diamine.

De préférence, dans la composition durcissable selon l'invention, l'accélérateur est choisi parmi les acides de Lewis, les amines tertiaires ou l'imidazole et ses dérivés.

Selon un mode de réalisation, dans la composition durcissable selon l'invention les accélérateurs et/ou durcisseurs sont directement incorporés dans la composition de prépolymères époxy. La composition durcissable selon l'invention est alors de type mono-composante (système 1K).

Selon un mode de réalisation, dans la composition durcissable selon l'invention les accélérateurs et/ou les durcisseurs sont conditionnés séparément de la composition de prépolymères époxy. La composition durcissable selon l'invention est alors de type bi-composante (système 2K).

Selon un autre aspect de la présente invention, il est proposé un polyépoxyde obtenu pas durcissement de la

composition durcissable selon l'invention.

Le durcissement (c'est-à-dire la réticulation) de la composition durcissable selon l'invention peut se faire spontanément ou bien nécessiter un chauffage ou une irradiation par un rayonnement UV.

En particulier, la composition durcissable selon l'invention peut être réticulée à une température comprise entre 5°C et 260°C.

Plus particulièrement, la composition durcissable selon l'invention peut être soumise à un cycle de cuisson comportant optionnellement une période à température ambiante suivie d'une ou plusieurs périodes de chauffage à des températures croissantes et comprises entre 30°C et 260°C. Par exemple, la composition durcissable selon l'invention peut être soumise à un cycle de cuisson de 1 heure à 80°C suivi de 2 heures à 180°C.

De préférence, le polyépoxyde selon l'invention présente une température de transition vitreuse (Tg) supérieure ou égale à 70°C, en particulier comprise entre 70°C et 210°C, plus particulièrement comprise entre 90°C et 200°C.

La température de transition vitreuse du polyépoxyde selon l'invention peut être déterminée par les techniques connues de l'homme du métier, notamment par calorimétrie différentielle à balayage (DSC), par exemple au moyen d'un appareil DSC Q20 dans un creuset ouvert avec un cycle Heat/Cool/Heat de 0°C à 200°C à 10°C/min, ou encore par analyse mécanique dynamique (DMA), par exemple au moyen d'un appareil Rhéomètre MCR 501 Anton Paar équipé de mors de torsion à une température régulée de 25°C à 250°C à 5°C/min et une fréquence de 1Hz.

Par ailleurs, le polyépoxyde selon l'invention présente une reprise en eau inférieure ou égale à 15%, en particulier comprise entre 0,1% et 11%, plus particulièrement entre 0,5% et 9,5%, préférentiellement entre 1% et 9%, plus particulièrement entre 1,5% et 8,5%, encore plus particulièrement entre 2% et 8%.

La reprise en eau du polyépoxyde est déterminée par mesure de la masse d'un échantillon avant et après saturation en eau obtenue par immersion dans l'eau à température ambiante pendant un temps suffisant. Par exemple, la reprise en eau du polyépoxyde peut être déterminée sur des échantillons parallélépipédique de 50 mm x 25 mm x 2 mm immergés pendant 96h dans l'eau à température ambiante, selon la formule suivante :

[Math. 1]

$$\text{reprise en eau } (\%) = \frac{(\text{masse après immersion} - \text{masse sèche})}{\text{masse sèche}}$$

Selon un autre aspect de la présente invention, il est proposé un matériau composite, revêtement ou adhésif comprenant le polyépoxyde selon l'invention.

Les matériaux composites selon l'invention peuvent être des matériaux composites de type polyépoxyde/fibres dont les fibres peuvent être notamment choisies parmi des fibres de verre, des fibres de carbones, des fibres basaltes, des fibres végétales (lin, chanvre).

Les matériaux composites selon l'invention peuvent être utiles pour la réalisation de pièces de structures performantes, comme par exemple dans le domaine de l'automobile, le domaine nautique le domaine aéronautique ou encore le domaine des sports et loisirs.

**Exemples**

La reprise en eau des échantillons est déterminée sur des échantillons parallélépipédique de 50 mm x 25 mm x 2 mm immergés pendant 96h dans l'eau à température ambiante, selon la formule suivante :

[Math. 2]

$$\text{reprise en eau } (\%) = \frac{(\text{masse après immersion} - \text{masse sèche})}{\text{masse sèche}}$$

Les températures de transition vitreuse (Tg) sont déterminées par calorimétrie différentielle à balayage (DSC) dans les conditions suivantes : Appareil : DSC Q20

Entre 10 et 20 mg de produit sont déposés dans un creuset ouvert. Un Cycle Heat/Cool/Heat est réalisé de 0°C à 200°C à 10°C/min.

Les viscosités sont mesurées à l'aide d'un viscosimètre rotatif Brookfield, type DV-II+. La mesure est effectuée après stabilisation du milieu maintenu à 25°C à l'aide d'un bain d'eau thermostaté. Les mesures de viscosité sont obtenues

avec un couple (Torque) en % du couple maximum du viscosimètre compris entre 10 et 100%.

L'équivalent époxy en poids (EEW) est mesuré suivant la norme ISO 3001 ou ASTM D1652.

Exemple 1 : isosorbide 100% (exemple comparatif)

Dans un réacteur double enveloppe d'une capacité de 2,5L, muni d'une pale d'agitation et d'un Dean Stark inversé surmonté d'un réfrigérant, on introduit 200g d'isosorbide, 633g d'épichlorhydrine (5 équivalents en mol par rapport au diol) ainsi que 2g de bromure de tetraethylammonium (TEAB, 1% en masse par rapport à la masse de diol). Le milieu de réaction est mis en chauffe (température de consigne : 110°C) sous un vide partiel, assuré par une pompe à palette, de 275 mbar (ce qui correspond à une dépression de 1013-275 = 738 mbar). Après distillation d'une quantité d'épichlorhydrine suffisante pour remplir le Dean-Stark inversé, on introduit à l'aide d'une pompe péristaltique 230g de solution aqueuse d'hydroxyde de sodium à 50% en masse sur une durée de 3 heures. Lors de l'addition de soude, la distillation de l'azéotrope eau-épichlorhydrine et la démixtion dans le Dean-Stark permettent de soustraire l'eau introduite et formée lors de la réaction. Une fois l'introduction de soude terminée, le milieu est laissé en chauffe et en distillation jusqu'à obtenir une température du milieu de 90°C. Arrivé à cette température, le chauffage est coupé et le milieu est laissé à refroidir à température ambiante. Le milieu est ensuite dépoté et les sels formés au cours de la réaction sont filtrés à l'aide d'un verre fritté de porosité 3. Le gâteau de sels est ensuite lavé à l'aide de 150g d'acétone. Le filtrat est récupéré. Les solvants de lavage et l'épichlorhydrine résiduelle sont éliminés par distillation sous vide à l'aide d'un évaporateur rotatif. On obtient 338g d'une huile visqueuse homogène jaune. Les résultats des analyses menées sur le prépolymère époxy obtenu sont présentés dans le Tableau 1.

[Tableau 1]

| | Exemple 1 | Exemple 2 | Exemple 3 |
|---|---|---|---|
| EEW (g/éq.) | 189 | 164 | 169 |
| Taux de conversion isosorbide | 100% | 99.3% | 99.6% |
| Taux de conversion CHDM | - | 91.1% | 79.6% |
| Viscosité (mPa.s) | 4360 | 175 | 990 |
| Teneur en eau (g/100g) | 0.15 | 0.02 | 0.05 |

Exemple 2 : isosorbide 25% / CHDM 75% (exemple selon l'invention)

Dans un réacteur double enveloppe d'une capacité de 500mL, muni d'une pale d'agitation et d'un Dean Stark inversé surmonté d'un réfrigérant, on introduit 10g d'isosorbide, 29.3g de CHDM, 126g d'épichlorhydrine (5 équivalents en mol par rapport au diol) ainsi que 400mg de bromure de tetraéthylammonium (TEAB, 1% en masse par rapport à la masse de diol). Le milieu de réaction est mis en chauffe (température de consigne : 110°C) sous un vide partiel, assuré par une pompe à palette, de 275 mbar (ce qui correspond à une dépression de 1013-275 = 738 mbar). Après distillation d'une quantité d'épichlorhydrine suffisante pour remplir le Dean-Stark inversé, on introduit à l'aide d'une pompe péristaltique 45g de solution aqueuse d'hydroxyde de sodium à 50% en masse sur une durée de 3 heures. Lors de l'addition de soude, la distillation de l'azéotrope eau-épichlorhydrine et la démixtion dans le Dean-Stark permettent de soustraire l'eau introduite et formée lors de la réaction. Une fois l'introduction de soude terminée, le milieu est laissé en chauffe et en distillation jusqu'à obtenir une température du milieu de 90°C. Arrivé à cette température, le chauffage est coupé et le milieu est laissé à refroidir à température ambiante. Le milieu est ensuite dépoté et les sels formés au cours de la réaction sont filtrés à l'aide d'un verre fritté de porosité 3. Le gâteau de sels est ensuite lavé à l'aide de 50g d'acétone. Le filtrat est récupéré. Les solvants de lavage et l'épichlorhydrine résiduelle sont éliminés par distillation sous vide à l'aide d'un évaporateur rotatif. On obtient 67g d'une huile visqueuse homogène jaune. Les résultats des analyses menées sur le prépolymère époxy obtenu sont présentés dans le Tableau 1.

Exemple 3 : isosorbide 75% / CHDM 25% (exemple selon l'invention)

Dans un réacteur double enveloppe d'une capacité de 2.5L, muni d'une pale d'agitation et d'un Dean Stark inversé surmonté d'un réfrigérant, on introduit 174.6g d'isosorbide, 58.2g de CHDM, 644g d'épichlorhydrine (5 équivalents en mol par rapport au diol) ainsi que 2.32g de bromure de tetraéthylammonium (TEAB, 1% en masse par rapport à la masse de diol). Le milieu de réaction est mis en chauffe (température de consigne : 110°C) sous un vide partiel assuré par une pompe a palette de 275 mbar (ce qui correspond à une dépression de 1013-275 = 738 mbar). Après distillation d'une quantité d'épichlorhydrine suffisante pour remplir le Dean-Stark inversé, on introduit à l'aide d'une pompe péristaltique

235g de solution aqueuse d'hydroxyde de sodium à 50% en masse sur une durée de 3 heures. Lors de l'addition, la distillation de l'azéotrope eau-épichlorhydrine et la démixtion dans le Dean-Stark permettent de soustraire l'eau introduite et formée lors de la réaction. Une fois l'introduction de soude terminée, le milieu est laissé en chauffe et en distillation jusqu'à obtenir une température du milieu de 90°C. Arrivé à cette température, le chauffage est coupé et le milieu est laissé à refroidir à température ambiante. Le milieu est ensuite dépoté et les sels formés au cours de la réaction sont filtrés à l'aide d'un verre fritté de porosité 3. Le gâteau de sels est ensuite lavé à l'aide de 150g d'acétone. Le filtrat est récupéré. Les solvants de lavage et l'épichlorhydrine résiduelle sont éliminés par distillation sous vide à l'aide d'un évaporateur rotatif. On obtient 352g d'une huile visqueuse homogène jaune. Les résultats des analyses menées sur le prépolymère époxy obtenu sont présentés dans le Tableau 1.

Les produits issus des exemples 2 et 3 (respectivement isosorbide 25%/CHDM 75% et isosorbide 75%/CHDM 25%) ont été réticulés avec de l'isophorone diamine (IPDA). Ainsi

Exemple 4 : 5 grammes du produit de l'exemple 2 ont été mélangés avec 1.30g d'IPDA avant de subir un cycle de cuisson de 1heure à 80°C suivi de 2 heures à 180°C.

Exemple 5 : 5 grammes du produit de l'exemple 3 ont été mélangés avec 1.27g d'IPDA avant de subir un cycle de cuisson de 1heure à 80°C suivi de 2 heures à 180°C

La température de transition vitreuse ainsi que la reprise en eau des produits réticulés ainsi obtenus ont été mesurées.

Les résultats sont présentés dans le tableau 2.

[Tableau 2]

| Polyépoxyde | Reprise en eau | Tg 2ème chauffage (°C) |
|---|---|---|
| Exemple 4 | 2.39% | 70 |
| Exemple 5 | 10.9% | 98 |
| Exemple 6 | 3% | 83 |
| Exemple 7 | 13% | 99 |
| Exemple 8 | 25% | 110 |

A titre comparatif, la réticulation de mélanges glycidyléther d'isosorbide/glycidyléther de CHDM a été effectuée. Ainsi

Exemple 6 : 5 grammes d'une composition comprenant 25% en mol de diglycidyléther d'isosorbide (EEW=189-g/eq) et 75% en mol de diglycidyléther de CHDM (EEW=159g/eq) ont été vigoureusement mélangés pendant 5 minutes avec 1.28g d'IPDA avant de subir en étuve un cycle de cuisson de 1heure à 80°C suivi de 2 heures à 180°C

Exemple 7 : 5 grammes d'une composition comprenant 75% en mol de diglycidyléther d'isosorbide (EEW=189-g/eq) et 25% en mol de diglycidyléther de CHDM (EEW=159g/eq) ont été vigoureusement mélangés pendant 5 minutes avec 1.17g d'IPDA avant de subir en étuve un cycle de cuisson de 1heure à 80°C suivi de 2 heures à 180°C.

La température de transition vitreuse ainsi que la reprise en eau des produits réticulés ainsi obtenus ont été mesurées.

Les résultats sont présentés dans le tableau 2.

La comparaison des matériaux selon l'invention et des matériaux réalisés à partir de mélanges de prépolymères époxy, montre que les premiers ont une meilleure tenue à l'eau (plus faible reprise en eau).

A titre comparatif, la réticulation d'un diglycidyléther d'isosorbide à l'aide d'IPDA a été effectuée. Ainsi

Exemple 8 : 5 grammes de diglycidyléther d'isosorbide (EEW=189g/eq) ont été vigoureusement mélangés pendant 5 minutes avec 1.12g d'IPDA avant de subir en étuve un cycle de cuisson de 1heure à 80°C suivi de 2 heures à 180°C

La comparaison des matériaux selon l'invention et du polyépoxyde obtenu à partir du diglycidyléther d'isosorbide seul montre que les premiers ont une tenue à l'eau nettement améliorée et une résistance thermique diminuée..

## Revendications

1. Procédé de préparation d'une composition de prépolymères époxy comprenant des glycidyléthers, ledit procédé comprenant les étapes suivantes :

   a) Mettre en contact un dianhydrohexitol et un autre alcool de manière à obtenir une composition d'alcools ;
   b) Faire réagir la composition d'alcools obtenue à l'étape a) avec une épihalohydrine de manière à obtenir un

mélange réactionnel comprenant des glycidyléthers ;
c) Récupérer la composition de prépolymères époxy comprenant des glycidyléthers à partir du mélange réactionnel obtenu à l'issue de l'étape b).

2. Procédé selon la revendication 1, **caractérisé en ce que** le dianhydrohexitol est un isohexitol, de préférence choisi parmi l'isosorbide, l'isomannide ou l'isoidide, et est, plus préférentiellement, l'isosorbide.

3. Procédé selon l'une quelconque des revendications précédentes, **caractérisé en ce que** l'autre alcool est choisi parmi les alcools suivants :

- Triméthylol éthane,
- Triméthylol propane,
- Spiroglycol,
- Tricyclodécanediméthanol,
- Glycérol,
- Hexan-1,6-diol,
- Diols aliphatiques en $C_n$, où $n \geq 7$,
- Cyclohexan-1,m-diméthanol, où m = 2, 3 ou 4,
- Furan-p,q-diméthanol, où {p,q} = {1,4}, {1,3} ou {2,3},
- Thiophen-p,q-diméthanol, où {p,q} = {1,4}, {1,3} ou {2,3},
- Isobornéol,
- Dodécanol, ou
- Décanol.

4. Procédé selon l'une quelconque des revendications précédentes, **caractérisé en ce que** l'autre alcool comprend au moins au moins deux fonctions alcool.

5. Procédé selon l'une quelconque des revendications précédentes, **caractérisé en ce que** un polyépoxyde obtenu par un procédé de durcissement d'une composition de prépolymères époxy obtenue par un procédé d'éthérification de l'autre alcool avec une épihalohydrine, a de préférence une reprise en eau inférieure à la reprise en eau, mesurée selon la même méthode, d'un polyépoxyde obtenu par les mêmes procédés de durcissement et d'éthérification mais en remplaçant l'autre alcool par le dianhydrohexitol.

6. Procédé selon l'une quelconque des revendications précédentes, **caractérisé en ce que** l'épihalohydrine est choisie parmi l'épibromohydrine, l'épifluorohydrine, l'épiiodohydrine, l'épichlorhydrine, ou leurs mélanges, de préférence l'épihalohydrine est l'épichlorhydrine.

7. Procédé selon l'une quelconque des revendications précédentes, dans lequel l'étape b) de réaction de la composition d'alcools avec l'épihalohydrine comprend les étapes suivantes :

b1) Mettre en contact la composition d'alcools et l'épihalohydrine de manière à obtenir un mélange réactionnel ;
b2) Placer le mélange réactionnel obtenu à l'étape b1) sous vide de manière à obtenir une dépression comprise entre 100mbar et 1000mbar ;
b3) Chauffer le mélange réactionnel obtenu à l'étape b2), en maintenant ladite dépression, à une température comprise entre 50°C et 120°C de manière à réaliser une distillation de l'épihalohydrine ;
b4) Ajouter au mélange réactionnel obtenu à l'étape b3) un réactif basique pendant une durée comprise entre 1h et 10h tout en maintenant le mélange réactionnel à ladite dépression et à ladite température de manière à réaliser une distillation azéotropique de l'azéotrope eau-épihalohydrine.

8. Procédé selon la revendication 7, **caractérisé en ce que** le réactif basique est choisi parmi les hydroxydes de lithium, de potassium, de calcium ou de sodium, de préférence sous forme d'une solution aqueuse, et est plus préférentiellement une solution aqueuse d'hydroxyde de sodium.

9. Procédé selon les revendications 7 ou 8, **caractérisé en ce qu'**un catalyseur de transfert de phase est ajouté lors de l'étape b1).

10. Procédé selon la revendication 9, **caractérisé en ce que** le catalyseur de transfert de phase est choisi parmi les halogénures, les sulfates ou les hydrogénosulfates de tétra-alkylammonium, de préférence parmi le bromure de

tétrabutylammonium ou l'iodure de tétrabutylammonium.

**11.** Procédé selon l'une quelconque des revendications 9 ou 10, caractérisé en ce la quantité de catalyseur de transfert de phase est comprise entre 0,01 et 5%, de préférence entre 0,1% et 2%, plus préférentiellement est de 1% en poids par rapport au total représenté par la somme des masses du dianhydrohexitol et de l'autre alcool.

**12.** Procédé selon l'une quelconque des revendications précédentes, **caractérisé en ce que** l'étape c) comprend une étape de filtration du milieu réactionnel obtenu à l'issue de l'étape b) de manière à obtenir un filtrat comprenant la composition de prépolymères époxy, de préférence ladite étape de filtration est suivie d'une étape de concentration du filtrat et/ou d'une étape de purification du filtrat.

**13.** Composition de prépolymères époxy susceptible d'être obtenue par le procédé selon l'une quelconque des revendications précédentes.

**14.** Composition durcissable comprenant la composition de prépolymères époxy selon la revendication 13, **caractérisée en ce qu'**elle comprend en outre au moins un accélérateur et/ou au moins un durcisseur, de préférence ladite composition durcissable comprend un durcisseur de type amine, plus préférentiellement le durcisseur est l'isophorone diamine.

**15.** Composition durcissable selon la revendication 14, **caractérisée en ce que** les accélérateurs et/ou durcisseurs sont directement incorporés dans la composition de prépolymères époxy.

**16.** Composition durcissable selon la revendication 15, **caractérisée en ce que** les accélérateurs et/ou les durcisseurs sont conditionnés séparément de la composition de prépolymères époxy.

**17.** Polyépoxyde obtenu par durcissement de la composition durcissable selon l'une quelconque des revendications 14 à 16.

**18.** Matériau composite, revêtement ou adhésif comprenant le polyépoxyde selon la revendication 17.

**Patentansprüche**

**1.** Ein Verfahren zur Herstellung einer Epoxidprepolymer-Zusammensetzung, die Glycidylether umfasst, wobei das Verfahren die folgenden Schritte umfasst:

a) In-Kontakt-Bringen eines Dianhydrohexitols mit einem weiteren Alkohol, um eine Zusammensetzung von Alkoholen zu erhalten;
b) Umsetzen der in Schritt a) erhaltenen Zusammensetzung von Alkoholen mit einem Epihalogenhydrin, um eine Glycidylether umfassende Reaktionsmischung zu erhalten;
c) Gewinnen der Glycidylether umfassenden Epoxidprepolymer-Zusammensetzung aus der am Ende von Schritt b) erhaltenen Reaktionsmischung.

**2.** Das Verfahren nach Anspruch 1, **dadurch gekennzeichnet, dass** das Dianhydrohexitol ein Isohexitol ist, vorzugsweise ausgewählt aus Isosorbid, Isomannid oder Isoidid, und besonders bevorzugt Isosorbid ist.

**3.** Das Verfahren nach einem der vorhergehenden Ansprüche, **dadurch gekennzeichnet, dass** der weitere Alkohol aus den folgenden Alkoholen ausgewählt ist:

◦ Trimethylolethan,
◦ Trimethylolpropan,
◦ Spiroglykol,
◦ Tricyclodecandimethanol,
◦ Glycerin,
◦ Hexan-1,6-diol,
◦ aliphatische $C_n$-Diolen, wobei $n \geq 7$,
◦ Cyclohexan-1, m-dimethanol, wobei m = 2, 3 oder 4,
◦ Furan-p,q-dimethanol, wobei {p,q\} = {1,4}, {1,3} oder {2,3},

◦ Thiophen-p,q-dimethanol, wobei {p,q} = {1,4}, {1,3} oder {2,3},
◦ Isoborneol,
◦ Dodecanol oder
◦ Decanol.

4. Das Verfahren nach einem der vorhergehenden Ansprüche, **dadurch gekennzeichnet, dass** der weitere Alkohol mindestens zwei Alkoholfunktionen umfasst.

5. Das Verfahren nach einem der vorhergehenden Ansprüche, **dadurch gekennzeichnet, dass** ein Polyepoxid, das durch ein Verfahren zum Härten einer Epoxidprepolymer-Zusammensetzung erhalten wurde, wobei diese durch ein Verfahren zur Veretherung des weiteren Alkohols mit einem Epihalogenhydrin erhalten wurde, vorzugsweise eine Wasseraufnahme aufweist, die geringer ist als die Wasseraufnahme, gemessen nach demselben Verfahren, eines Polyepoxids, das durch dieselben Härtungs- und Veretherungsverfahren erhalten wurde, jedoch durch Ersetzen des weiteren Alkohols durch Dianhydrohexitol.

6. Das Verfahren nach einem der vorhergehenden Ansprüche, **dadurch gekennzeichnet, dass** das Epihalogenhydrin ausgewählt ist aus Epibromhydrin, Epifluorhydrin, Epiiodhydrin, Epichlorhydrin oder Mischungen davon, wobei das Epihalogenhydrin vorzugsweise Epichlorhydrin ist.

7. Das Verfahren nach einem der vorhergehenden Ansprüche, wobei Schritt b) des Umsetzens der Zusammensetzung von Alkoholen mit Epihalogenhydrin die folgenden Schritte umfasst:

   b1) In-Kontakt-Bringen der Zusammensetzung von Alkoholen mit Epihalogenhydrin, um eine Reaktionsmischung zu erhalten;
   b2) Setzen der in Schritt b1) erhaltenen Reaktionsmischung unter Vakuum, um einen Unterdruck zwischen 100 mbar und 1.000 mbar zu erhalten;
   b3) Erhitzen der in Schritt b2) erhaltenen Reaktionsmischung unter Beibehaltung des Unterdrucks auf eine Temperatur zwischen 50°C und 120°C, um eine Destillation des Epihalogenhydrats zu erreichen;
   b4) Zugabe eines basischen Reagens zur in Schritt b3) erhaltenen Reaktionsmischung für einen Zeitraum zwischen 1 Stunde und 10 Stunden unter Beibehaltung des Unterdrucks und der Temperatur, um eine azeotrope Destillation des Wasser-Epihalogenhydrin-Azeotrops zu erreichen.

8. Das Verfahren nach Anspruch 7, **dadurch gekennzeichnet, dass** das basische Reagens ausgewählt ist aus Lithium-, Kalium-, Calcium- oder Natriumhydroxiden, vorzugsweise in Form einer wässrigen Lösung, und besonders bevorzugt eine wässrige Natriumhydroxidlösung ist.

9. Das Verfahren nach Anspruch 7 oder 8, **dadurch gekennzeichnet, dass** während Schritt b1) ein Phasentransferkatalysator zugegeben wird.

10. Das Verfahren nach Anspruch 9, **dadurch gekennzeichnet, dass** der Phasentransferkatalysator ausgewählt ist aus Halogeniden, Tetraalkylammoniumsulfaten oder -hydrogensulfaten, vorzugsweise aus Tetrabutylammoniumbromid oder Tetrabutylammoniumiodid.

11. Das Verfahren nach einem der Ansprüche 9 oder 10, **dadurch gekennzeichnet, dass** die Menge des Phasentransferkatalysators zwischen 0,01 und 5 %, vorzugsweise zwischen 0,1 % und 2 %, besonders bevorzugt bei 1 Gew.-% liegt, bezogen auf die Summe der Massen des Dianhydrohexitols und des weiteren Alkohols.

12. Das Verfahren nach einem der vorhergehenden Ansprüche, **dadurch gekennzeichnet, dass** Schritt c) einen Schritt des Filtrierens des am Ende von Schritt b) erhaltenen Reaktionsmediums umfasst, um ein Filtrat zu erhalten, das die Epoxidprepolymer-Zusammensetzung umfasst, wobei dem Filtrationsschritt vorzugsweise ein Schritt des Konzentrierens und/oder ein Schritt des Reinigens des Filtrats folgt.

13. Eine Epoxidprepolymer-Zusammensetzung, die durch das Verfahren nach einem der vorhergehenden Ansprüche erhältlich ist.

14. Eine härtbare Zusammensetzung, umfassend die Epoxidprepolymer-Zusammensetzung nach Anspruch 13, **dadurch gekennzeichnet, dass** sie ferner mindestens einen Beschleuniger und/oder mindestens ein Härtungsmittel umfasst, wobei die härtbare Zusammensetzung vorzugsweise ein Härtungsmittel vom Amintyp umfasst, besonders

bevorzugt ist das Härtungsmittel Isophorondiamin.

**15.** Die härtbare Zusammensetzung nach Anspruch 14, **dadurch gekennzeichnet, dass** die Beschleuniger und/oder Härtungsmittel direkt in die Epoxidprepolymer-Zusammensetzung eingearbeitet sind.

**16.** Die härtbare Zusammensetzung nach Anspruch 15, **dadurch gekennzeichnet, dass** die Beschleuniger und/oder Härtungsmittel getrennt von der Epoxidprepolymer-Zusammensetzung verpackt sind.

**17.** Ein Polyepoxid, erhalten durch Härten der härtbaren Zusammensetzung nach einem der Ansprüche 14 bis 16.

**18.** Ein Verbundstoff-, Beschichtungs- oder Klebematerial, welches das Polyepoxid nach Anspruch 17 umfasst.

## Claims

**1.** A method for preparing an epoxy prepolymer composition comprising glycidyl ethers, said method comprising the following steps:

a) Placing a dianhydrohexitol into contact with another alcohol so as to obtain a composition of alcohols;
b) Reacting the composition of alcohols obtained in step a) with an epihalohydrin so as to obtain a reaction mixture comprising glycidyl ethers;
c) Recovering the epoxy prepolymer composition comprising glycidyl ethers from the reaction mixture obtained at the end of step b).

**2.** The method according to claim 1, **characterized in that** the dianhydrohexitol is an isohexitol, preferably selected from isosorbide, isomannide or isoidide, and is, more preferably, isosorbide.

**3.** The method according to any one of the preceding claims, **characterized in that** the other alcohol is selected from the following alcohols:

- Trimethylol ethane,
- Trimethylol propane,
- Spiroglycol,
- Tricyclodecanedimethanol,
- Glycerol,
- Hexan-1,6-diol,
- $C_n$ aliphatic diols, wherein $n \geq 7$,
- Cyclohexan-1, m-dimethanol, where m = 2, 3 or 4,
- Furan-p,q-dimethanol, where {p,q} = {1,4}, {1,3} or {2,3},
- Thiophen-p,q-dimethanol, where {p,q} = {1,4}, {1,3} or {2,3},
- Isoborneol,
- Dodecanol, or
- Decanol.

**4.** The method according to any one of the preceding claims, **characterized in that** the other alcohol comprises at least two alcohol functions.

**5.** The method according to any one of the preceding claims, **characterized in that** a polyepoxide obtained by a method for curing an epoxy prepolymer composition obtained by a method for the etherification of the other alcohol with an epihalohydrin, preferably has a water uptake lower than the water uptake, measured according to the same method, of a polyepoxide obtained by the same curing and etherification methods but by replacing the other alcohol with dianhydrohexitol.

**6.** The method according to any one of the preceding claims, **characterized in that** the epihalohydrin is selected from epibromohydrin, epifluorohydrin, epiiodohydrin, epichlorhydrin, or mixtures thereof, preferably epihalohydrin is epichlorhydrin.

**7.** The method according to any one of the preceding claims, wherein step b) of reacting the composition of alcohols with

epihalohydrin comprises the following steps:

b1) Placing the composition of alcohols into contact with epihalohydrin so as to obtain a reaction mixture;
b2) Placing the reaction mixture obtained in step b1) under vacuum so as to obtain a negative pressure comprised between 100 mbar and 1,000 mbar;
b3) Heating the reaction mixture obtained in step b2), while maintaining said negative pressure, at a temperature comprised between 50°C and 120°C, so as to achieve distillation of the epihalohydrate;
b4) Adding a basic reagent to the reaction mixture obtained in step b3) for a period comprised between 1 hour and 10 hours while maintaining the reaction mixture at said negative pressure and at said temperature so as to achieve azeotropic distillation of the water-epihalohydrin azeotrope.

**8.** The method according to claim 7, **characterized in that** the basic reagent is selected from lithium, potassium, calcium or sodium hydroxides, preferably in the form of an aqueous solution, and is more preferably an aqueous sodium hydroxide solution.

**9.** The method according to claims 7 or 8, **characterized in that** a phase-transfer catalyst is added during step b1).

**10.** The method according to claim 9, **characterized in that** the phase-transfer catalyst is selected from halides, tetraalkyl ammonium sulfates or hydrogen sulfates, preferably from tetrabutylammonium bromide or tetrabutylammonium iodide.

**11.** The method according to any one of claims 9 or 10, **characterized in that** the amount of phase-transfer catalyst is comprised between 0.01 and 5%, preferably between 0.1% and 2%, more preferably is 1% by weight relative to the total represented by the sum of the masses of the dianhydrohexitol and of the other alcohol.

**12.** The method according to any one of the preceding claims, **characterized in that** step c) comprises a step of filtering the reaction medium obtained at the end of step b) so as to obtain a filtrate comprising the epoxy prepolymer composition, preferably said filtration step is followed by a step of concentrating the filtrate and/or a step of purifying the filtrate.

**13.** An epoxy prepolymer composition that can be obtained by the method according to any one of the preceding claims.

**14.** A curable composition comprising the epoxy prepolymer composition according to claim 13, **characterized in that** it further comprises at least one accelerating agent and/or at least one curing agent, preferably said curable composition comprises an amine-type curing agent, more preferentially the curing agent is isophorone diamine.

**15.** The curable composition according to claim 14, **characterized in that** the accelerating agents and/or curing agents are directly incorporated into the epoxy prepolymer composition.

**16.** The curable composition according to claim 15, **characterized in that** the accelerating agents and/or the curing agents are packaged separately from the epoxy prepolymer composition.

**17.** A polyepoxide obtained by curing the curable composition according to any one of claims 14 to 16.

**18.** A composite, coating or adhesive material comprising the polyepoxide according to claim 17.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 3272845 A
- US 4770871 A
- WO 2008147472 A
- WO 2008147473 A
- US 3041300 A
- WO 2012157832 A
- WO 2015110758 A
- US 20150353676 A1
- US 20180230261 A1
- WO 2015110758 A1
- US 20170253692 A
- JP 2014189713 B